# EUROPEAN PATENT APPLICATION

(11) **EP 4 467 981 A1**
(43) Date of publication of application: **27.11.2024**
(21) Application number: 24177962.8
(22) Date of filing: 24.05.2024
(51) Int. Cl.: G01N 33/543, G01N 21/64, G01N 21/03, G01N 21/77, G01N 21/84, G01N 1/31, G01N 35/00, G01N 1/30, G02B 21/34

(54) **METHOD FOR GENERATING AT LEAST A FIRST CONTROL AREA AND MEANS AND A METHOD FOR ANALYSING A BIOLOGICAL SAMPLE**

(30) Priority: 26.05.2023 EP 23175688
(71) Applicant: Leica Microsystems CMS GmbH, 35578 Wetzlar (DE)
(72) Inventor: Schlaudraff, Falk, 35578 Wetzlar (DE); Schlicker, Oliver, 35578 Wetzlar (DE); Alsheimer, Soeren, 35578 Wetzlar (DE)
(74) Representative: Schaumburg und Partner Patentanwälte mbB

(57) **Abstract**

In one aspect, a method for generating at least a first control area (102) for a biological analysis is provided. The method comprises: depositing on a surface a solution comprising a first control analyte (200a, 200b, 200c, 200d) in order to generate the first control area (102). Further, a system (500) for generating at least the first control area (102) is provided as well as means comprising the control area (102) and a method for analysing a biological sample (106).

## Description

### Technical field

The invention relates to a method for generating at least a first control area for a biological analysis. Further, a system for generating at least the first control area is provided as well as means comprising the control area and a method for analysing a biological sample.

### Background

In the art, systems are known for analysing biological samples such as tissue sections to determine the spatial distribution of target analytes within the biological samples. This generally relies on staining with optically detectable, in particular fluorescent, markers that are specific to the respective analytes. The biological samples are often provided on sample holders, such as microscope slides, in order to simplify handling of a large number of individual biological samples.

A particularly important aspect of this analysis relies on successfully staining these samples repeatedly. Thus, when analysing the biological samples by means of staining with markers, it is often desirable to be able to check if the staining procedure was successful or not. In particular when analysing a large number of biological samples, it is desirable to be able to check if the staining procedure was successful or not particularly efficiently. Moreover, in case collections of large numbers of biological samples are already provided on means for storing or handling these collections such as glass slides, for example, it is desirable to nevertheless be able to check and verify staining procedures without having to rearrange the biological samples to dedicated analysis means.

### Summary

It is an object to provide methods and means that enable efficiently and flexibly verifying of staining procedures, in particular for existing collections of biological samples.

The aforementioned object is achieved by the subject-matter of the independent claims. Advantageous embodiments are defined in the dependent claims and the following description.

In one aspect, a method for generating at least a first control area for a biological analysis is provided. The method comprises: depositing on a surface a solution comprising a first control analyte in order to generate the first control area. The first control area might be arranged spaced apart from or adjacent to a biological sample for which the biological analysis is carried out. In other words, the first control area and the biological sample are arranged separately from each other. Thus, the first control area comprises the first control analyte. This enables flexibly generating control areas on means to store or handle collections of biological samples. In particular, the solution is deposited in the first control area. After the solution is deposited on the surface, the solvent of the solution may evaporate and the control analyte as the solute may remain in the first control area on the surface. The solution is preferably a liquid, in particular an aqueous solution. The solution preferably has a predetermined concentration of the control analyte. For example, the first control analyte is configured to bind a first marker, such as an antibody, during a staining procedure of a biological sample.

Preferably, a polymerising reagent is deposited in the first control area. This enables generating a particularly robust first control area. In particular, the polymerising agent is deposited on the surface at a position of the first control area. The polymerising reagent may be in a solution comprising the polymerising reagent. For example, the polymerising reagent may cross-link the control analyte or cause cross-linking of the control analyte. Further, the polymerising reagent may link the control analyte to the surface. The polymerising reagent may be deposited prior to, subsequent to or simultaneously with the solution comprising the at least one control analyte.

Preferably, the control analyte or polymerising solution is deposited on the surface in form of at least one droplet. This enables a particularly accurate placement and deposition of the solution, whilst efficiently generating a large number of control areas. Further, this enables contactless deposition of the solution on the surface. In particular, the droplet has a volume in a range of 1 to 1000 nanolitres. The droplet may be dispensed or generated by means of a piezo actuated dispenser.

Preferably, the solution comprises the first control analyte at a predetermined density.

The control analyte may, for example, be identical to a target analyte to be detected in a biological sample. Thus, a marker configured to bind to the target analyte would consequently also bind to the control analyte. In a further alternative example, the control analyte may be configured to bind to a class of markers and each marker may bind to a different target analyte. For example, the control analyte may bind to an Fc-fragment of a particular type of antibody. This control analyte may therefore bind to any marker that is based on that particular type of antibody, irrespective of the particular target analyte the markers may bind.

Preferably, the first control analyte is a biomolecule. This enables efficiently capturing markers that are configured for biological analysis. In particular, biomolecules may include large macromolecules such as proteins, carbohydrates, lipids, and nucleic acids, as well as small molecules such as primary metabolites, secondary metabolites and natural products. Thus, a biomolecule may be biological material. Generally, a biomolecule may be an endogenous molecule. Preferably the biomolecule is a protein or a nucleic acid. Alternatively, the control analyte may be configured to bind antibodies, for example to a Fc-fragment. Thus, the control analyte may be configured to bind antibody-based markers.

Preferably, a second solution comprising a second control analyte is deposited on the surface, and in particular in or near or adjacent to the first control area. This enables efficiently generating a control area for a biological analysis comprising several different markers. In particular, the second control analyte is deposited either within the first control area, or within a second control area. Similarly, further control analytes may be used or further control areas may be generated. Alternatively, the solution of the first control analyte may comprise the second control analyte. Preferably each solution only comprises a single type of control analyte. This enables specifically providing control areas with specific control analytes that depend on a particular biological sample.

Preferably, the surface is of a base of a sample holder. The base may be a rigid base, such as a glass slide or a microscope slide, for example. In particular, the solution comprising the control analyte is deposited on the surface of the base of the sample holder. This enables efficiently generating the control area on the base of the sample holder.

It is particularly preferred, that the base of the sample holder comprises a sample area, which is configured to receive a biological sample, or wherein the base of the sample holder comprises a sample area with a biological sample, and the first control analyte is identical to a first target analyte of the biological sample. Thus, the sample area is configured to hold the biological sample comprising the first target analyte. This enables efficient biological analysis of the biological sample based on the first control analyte in the first control area. In particular, in case of a large collection of existing biological samples, each on a sample holder, the method enables efficiently generating control areas on the base after the biological samples have been arranged in the sample area of the sample holder. It is particularly preferred that the control analyte to be deposited in the first control area on the base of the sample holder is determined based on the biological sample in the sample area of the sample holder, in particular, based on the target analyte of the biological sample.

Preferably, the base of the sample holder comprises a sample area with a biological sample and the biological sample, in particular its location or its outline, is determined on the base of the sample holder and the solution comprising the first control analyte is deposited on the surface of the base adjacent to the biological sample. This enables generating the first control area precisely and in a predetermined distance from the biological sample or a predetermined location on the base relative to the biological sample. The biological sample may be determined on the base of the sample holder by generating an optical readout of the base and determine in the optical readout data the biological sample, for example by segmentation analysis. The control area is generally deposited on the base after the biological sample.

Preferably, the surface is of a flexible carrier with a first side and a second side opposite the first side. The solution comprising the first control analyte is deposited on the first side of the flexible carrier and the second side comprises an adhesive. Thus, the flexible carrier may be an adhesive strip, for example. The generated flexible carrier enables flexible application of the control area to further devices. Thus, the method enables flexible application of the control area. The method may comprise an additional step of depositing the adhesive on the second side of the flexible carrier.

Preferably, the flexible carrier is fixed to a base of a sample holder. In particular, the second side of the flexible carrier may be fixed to the base by the adhesive. This enables generating the control area on the sample holder.

In a further aspect, a system for generating at least a first control area comprising at least a first control analyte on a surface is provided. The system comprises means configured to carry out the method for generating at least a first control area. The system preferably includes a liquid reservoir for at least the solution comprising the first control analyte and a liquid dispenser for dispensing the solution from the liquid reservoir onto the surface. The liquid reservoir may be a multititer plate, for example, with each well holding a solution with a different control analyte. The liquid dispenser may be a contactless droplet dispenser, for example, such as an ultrasonic or a piezo dispenser. The liquid dispenser is configured to dispense droplets of a volume in a range of 1 to 1000 nanolitres, for example. The system enables efficiently generating a large number of control areas, for example, on a surface of a base of a sample holder.

In a further aspect, a flexible carrier for carrying at least a first control area is provided. The flexible carrier comprises a first side and a second side opposite the first side, wherein the first side of the carrier comprises at least a first control area comprising at least a first control analyte. The second side of the carrier comprises an adhesive. Thus, the flexible carrier may be an adhesive strip, for example. The generated flexible carrier enables flexible application of the control area to further devices. In particular, the control area on the flexible carrier may be generated by the method for generating at least a first control area, for example, according to one of the claims 1 to 5 and 9 to 10.

In a further aspect, a sample holder for a biological sample is provided, the biological sample including at least one first target analyte. The sample holder comprises a sample area configured to hold the biological sample, and at least a first control area comprising at least one first control analyte identical to the one first target analyte. In particular, the sample holder may be used during staining of the biological sample and during generating an optical readout of the biological sample. The sample holder enables controlling or verifying if during staining of the biological sample provided on the sample holder, optically detectable markers have successfully bound to the control analyte and therefore bound to the target analyte in the biological sample. The optically detectable markers may be fluorescent markers comprising an affinity reagent specific to the first target analyte and the first control analyte and at least one fluorescent dye, for example. Preferably, the first control area of the sample holder is generated by the method for generating at least a first control area, in particular, the method according to one of the claims 1 to 8.

The first control analyte may be an oligonucleotide or protein directly attached to the first control area, for example. Further, the first control analyte may also be within a cell or a tissue attached to the first control area.

The first control area is preferably arranged at a fixed and/or predetermined location of the sample holder. This enables efficient repetitive analysis of the sample holder, in particular of a plurality of sample holders.

Preferably, the at least first control area comprises the first control analyte at a predetermined density. This means, that the first control analyte is arranged in the first control area at a known quantity per area of the control area. Preferably, the distribution of the first control analyte in the first control area is homogeneous. This enables determining the emission intensity of specific markers bound to the first control analyte in relation to their quantity bound to the first control analyte, e.g. the emission intensity of fluorescent light being emitted by excited fluorescent molecules or fluorescent dyes of at least one specific marker. Alternatively or additionally, several first control areas may be provided, for example, each first control area may comprise the first control analyte at a particular predetermined density different to the densities of the remaining first control areas. This enables determining the emission intensity of specific markers bound to the first control analyte over a range of densities.

Preferably, the sample holder comprises an identifier. The identifier enables identification of a particular one of the sample holders in a plurality of sample holders. The identifier might comprise an RFID.

It is particularly preferred, that the identifier is optically detectable, e.g. in the form of a barcode or a QR-code. This enables reading the identifier when generating an optical readout of the sample holder.

Preferably, the sample area is spatially separate from at least the first control area. This enables generating separate readouts from the sample area and the first control area. Additionally, the sample area and/or the control area may also be separate from the identifier.

Preferably, the sample holder comprises a base on which the sample area and the first control area are arranged and wherein the base is adapted to enable observation of the biological sample by means of an optical readout device, for example, a microscope. This enables particularly efficient handling of the sample holder and observation of the biological sample.

Preferably, at least a part of the sample holder comprising the sample area is optically transparent. In particular, the base is a planar glass slide, or a microscope slide. This enables particularly efficient handling of the sample holder and enables readily producing the sample holder.

Preferably, the at least first control area comprises at least one second control analyte, wherein, in particular, the at least one second control analyte is different from the at least one first control analyte. In particular, the second control analyte is identical to a second target analyte in the biological sample. This enables verifying the staining for at least a second control analyte.

Preferably, the sample holder comprises at least one second control area comprising at least one second control analyte, wherein the first control area is spatially separated from the second control area. This enables verifying the staining for at least a second control analyte. In particular, one of the control areas only comprises a single species of the control analytes. This enables verifying the staining for each control analyte independently, in particular without crosstalk between control analytes of different species.

In a further aspect, a method is provided for analysing a biological sample including at least one first target analyte. The biological sample is provided in the sample area of a sample holder, preferably a sample holder according to one of the claims 18 to 23. In particular, the method is used for analysing the staining of the biological sample including at least one first target analyte. The method comprises the following steps: step a): adding a staining reagent, at least comprising a first optically detectable marker specific to the first target analyte, to the sample area and to at least the first control area of the sample holder. Further, the method comprises step b): generating an optical readout of at least the first control area by means of an optical readout device, such as a microscope. Moreover, the method comprises step c): determining for at least the first control area an intensity parameter of at least the first optically detectable marker in the optical readout.

By adding the staining reagent to the sample area and the control area, the first marker may bind to the first target analyte and the first control analyte, respectively. In particular, the first target analyte is identical to the first control analyte. The first marker may therefore comprise an affinity reagent specific to the first target analyte and the first control analyte as well as a fluorescent label. The intensity parameter may be based on the intensity of the fluorescent emission light of the first marker. This enables determining based on the intensity parameter, if the staining of the biological sample and the control area was successful at all. For example, if the first optically detectable marker may not be detected in the optical readout, the staining may be determined as unsuccessful. Therefore, the intensity parameter can have a binary format, e.g. the staining was successful or it was unsuccessful. In this case, it can be encoded or stored as 1 or 0. Furthermore, the intensity parameter can depend on the amount of detected fluorescent emission light and can comprise a value being in the range between 0 and a maximum intensity value.

Preferably, the method further comprises the step d): generating at least one optical readout of the biological sample in the sample area and analysing the biological sample based on the determined intensity parameter. In particular the step d) follows the step c). This enables accurate analysis of the stained biological sample.

Preferably, the step c) or the step d) comprises rejecting the stained biological sample in case that the intensity parameter is below a predetermined threshold or has a value being 0. This enables efficient analysis of the stained biological sample.

Preferably, the first control area comprises the first control analyte at a predetermined density, and wherein step c) comprises adjusting at least one optical readout parameter of the optical readout device based on the intensity parameter. This enables accurate analysis of the biological sample.

Preferably in a step e) at least the first optically detectable marker is removed or quenched. This enables repeating the staining of the biological sample.

Preferably, the steps a) to e) are iteratively repeated. For example, different optically detectable markers may be used in each iteration. This enables detecting a plurality of target analytes in the biological sample. In one embodiment, individual control areas may be used for each round of staining.

Preferably, the method further comprises a step f): generating the first control area according to the method for generating at least a first control area, in particular according to one of the claims 1 to 10. Preferably, the step f) is carried out prior to step a). The first control area may be generated on a base of the sample holder. Step f) may further include generating an optical readout prior to generating the first control area in order to determine the location of the biological sample on the base of the sample holder. Further, the first control area may be generated on a sample holder by applying the flexible carrier with the first control area onto the sample holder. In particular, the second side of the flexible carrier may be placed on a base of the sample holder and the flexible carrier adheres by means of the adhesive of the second side on the base of the sample holder.

In a further aspect, an analysing system for analysing a biological sample comprising at least one first target analyte, configured to perform the method for analysing a biological sample is provided.

### Short Description of the Figures

Hereinafter, specific embodiments are described referring to the drawings, wherein:
- Figure 1: is a schematic view of a sample holder with a control area,
- Figure 2: is a schematic view of the control area with various control analytes, and
- Figure 3: is a flow diagram for a method to analyse a biological sample,
- Figure 4: is a schematic view of a flexible carrier for carrying a control area and a sample holder comprising the flexible carrier, and
- Figure 5: is a schematic view of a system for generating a first control area.

### Detailed Description

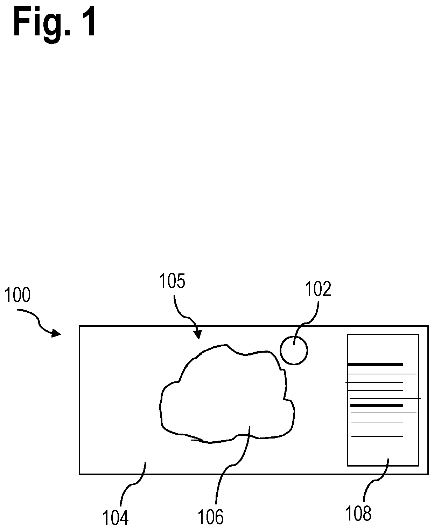

Figure 1 is a schematic view of a sample holder 100 with a control area 102. The sample holder comprises a base 104. Preferably, the base 104 is configured to enable observation of a biological sample 106, such as a tissue section, by means of an optical readout device. The optical readout device may be a microscope, a light microscope, a fluorescence light microscope, a widefield microscope, a scanning microscope, a confocal microscope, or an electron microscope, for example. The base 104 is preferably a microscope slide, for example, a planar glass slide. The control area 102 and the biological sample 106 within a sample area 105 are arranged on one side of the base 104. Thus, the control area 102 and/or the sample area 105 may be illuminated with illumination light from one side of the base 104 and observed by means of the microscope from the other side of the base 104, for example.

The control area 102 comprises at least one control analyte. The control analyte may be attached to the base 104 in the control area 102. The control analyte is, in particular, identical to a target analyte in the biological sample 106. This means, that the control analyte is of the same species as the target analyte, for example, the control analyte is the same protein as the target analyte. The sample holder 100 may comprise several control areas 102. In this case, each control area 102 may comprise a specific control analyte and/or each control area 102 may comprise the same control analyte but in different densities. Alternatively, each control area may comprise several different control analytes.

For example, the target analyte(s) of the biological sample 106 may be stained with optically detectable markers on the sample holder 100. In particular, at least one target analyte may be stained with a marker, the marker comprising an affinity reagent for specifically attaching the target analyte and a label for optically detecting the marker. The affinity reagent may be an antibody, for example and the label may be a fluorophore. Since the control analyte is the same as the target analyte in the biological sample, the marker binds to the target analyte as well as the control analyte. Thus, when staining the biological sample 106, the control area 102 of the sample holder 100 may be stained at the same time. This enables checking, if the staining procedure is successful or not, for example.

Preferably, the sample holder comprises an identifier 108, arranged on the base 104. The identifier 108 may be an optically detectable barcode, for example. Alternatively or in addition, the identifier 108 may be detectable by other means, for example, the identifier 108 may comprise a RFID-chip. The identifier 108 is particularly useful, when a plurality of different biological samples 106 are provided, each on one of the sample holders 100. By having a unique identifier 108 on each sample holder 100, the sample holders 100 and therefore the respective biological samples 106 can be identified individually.

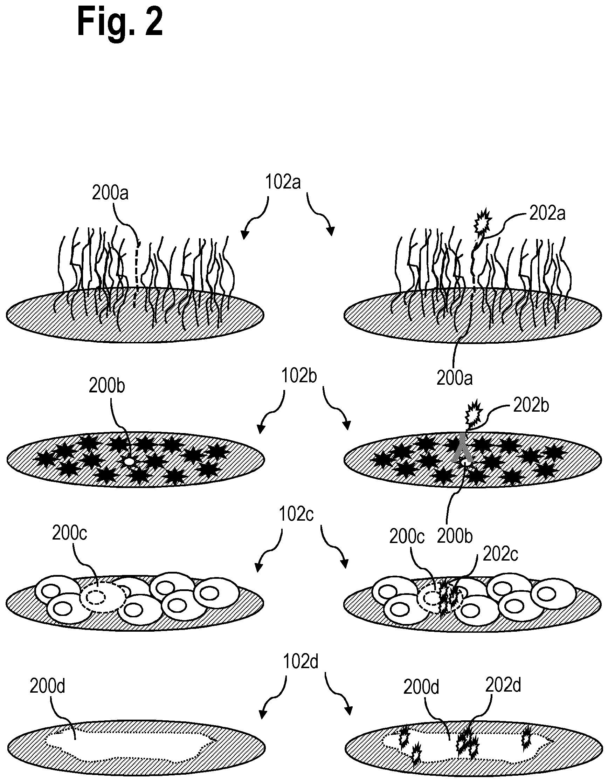

Figure 2 is a schematic view of embodiments of the control area 102 with different control analytes 200a, 200b, 200c, 200d. The control area 102a comprises oligonucleotides 200a as the control analyte. The oligonucleotide 200a may bind to a marker 202a comprising a complementary oligonucleotide as an affinity reagent.

The control area 102b comprises proteins 200b as the control analyte. The protein 200b may bind to markers 202b comprising an antibody specific to the protein 200b as an affinity reagent.

The control area 102c comprises cells 200c as the control analyte. The cell 200c may comprise an analyte, such as a protein, that markers 202c comprising an antibody specifically bind to.

The control area 102d comprises a tissue section 200d as the control analyte. The tissue section 200d may comprise an analyte, such as an oligonucleotide, that markers 202d comprising a complementary oligonucleotide bind to.

By applying markers 202a-202d for staining the biological sample 106, in particularly by binding specifically to the target analyte of the biological sample 106, to the sample area 105 and the control area 102 of the sample holder 100, the markers 202a-202d may bind to both, the target analyte and the respective control analyte 200a-200d. Thus, a successful staining may be determined by observing if the marker 202a-202d bound to the control analyte 200a-200d in the control area 102a-102d.

In particular in the case of the control analytes 200a, 200b the control analytes may be attached to the control area 102a, 102b in predetermined quantities.

In particular for the control analytes 200a, 200b in the control areas 102a, 102b, the control analytes 200a, 200b may be attached at predetermined densities, that means at predetermined and uniform amounts of control analyte 200a, 200b per unit area of the control area 102a, 102b.

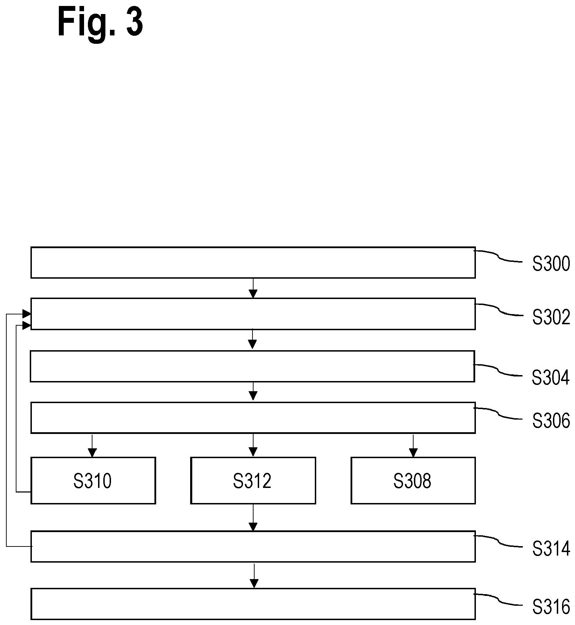

Figure 3 is a flow diagram for a method to analyse the biological sample 106, in particular, to analyse the staining of the biological sample 106. The biological sample 106 is provided in the sample area 105 of the sample holder 100. The method starts in step 5300. In step S302, a staining reagent is added to the sample area 105 and the control area 102. The staining reagent comprises optically detectable markers specific to a particular target analyte in the biological sample 106 and the respective control analyte 200a-200d in the control area 102a-102d. The biological sample 106 may comprise a plurality of target analytes, in which case the staining reagent may comprise plurality of markers 202a-202d that are each specific to a particular one of the target analytes. Similarly, the control area 102a-102d may comprise a plurality of respective control analytes 200a-200d. Alternatively, several control areas 102a-102d may be provided, each comprising a particular one of the control analytes 200a-200d. By applying the staining reagent, the markers 202a-202d bind to the respective target analytes and control analytes.

In a step S304, an optical readout of at least the control area 102 by means of an optical readout device. For example, the control area 102 may be imaged by means of a microscope.

In a step S306, an intensity parameter is determined for at least the first control area 102 in the optical readout of step S304. In case the optically detectable marker 202a-202d comprises fluorophores as labels, the intensity parameter may be the intensity of a fluorescent emission at an emission wavelength of the fluorophores, for example. The method ends in step S308, in case no intensity is detected and the intensity parameter is zero. In this case, the staining may be determined as unsuccessful.

Alternatively, the method may continue in a step S310, in case no intensity is detected and the intensity parameter is zero, in which case the method and the staining is repeated by starting with step S302.

Alternatively, the method may continue in step S312, if an intensity is detected, in particular, if the intensity parameter is above a predetermined threshold. Optionally, the step S312 may comprise adjusting the readout device based on the intensity parameter. For example, an excitation light intensity of the readout device, a spectral detection range of the readout device, or a detection duration or exposure time of the readout device may be adjusted. In a specific example, in case markers with green fluorescent protein (GFP) or red fluorescent protein (RFP) are used, the excitation light intensity may be adjusted for each, GFP and RFP, individually. Further, spectral unmixing parameters may be adjusted. Particularly, two makers with different labels that emit fluorescent light with overlapping wavelengths may be detected individually and separately by providing control areas that comprise control analytes to which either one marker binds. This enables determining the particular emission wavelengths for each marker individually.

In step S314, an optical readout of the sample area 105 with the biological sample 106 is generated. The biological sample 106, in particular the respective optical readout, may then be analysed based on the determined intensity parameter. For example, based on the intensity parameter, a quantitative analysis of the target analyte in the biological sample 106 may be carried out, in particular, when the respective control analyte 200a-200d is attached at a predetermined density. The method ends in step S316.

Alternatively, at least the biological sample 106 may be treated in order to remove or quench the markers bound to the biological sample 106 and the method may continue in step S302 with another staining cycle. This may continue iteratively, in order to stain the biological sample 106 with different markers in each staining cycle. Dedicated control areas 102 for each staining cycle and the respective control analytes 200a-200d may be provided on the sample holder 100.

An additional example of analysing the biological sample 106 based on the intensity parameter in step S314 relies on determining the intensity parameter of the markers without crosstalk. This may be achieved by providing a plurality of control areas 102, each with a single control analyte 200a-200d, such that when determining the intensity parameter in the readout of the control area 102 in step S306, the intensity parameter is determined without crosstalk to further markers. The intensity parameter determined in such a fashion may be used for unmixing of emission light from the markers in the optical readout generated in step S314. This is particularly useful in case several markers are used to detect a corresponding several target analytes in the biological sample 106.

In a further alternative, the staining reagent comprising the markers 202a-202d may be applied to the control area 102 together with binding agents unspecific to the respective control analyte, in order to determine the intensity parameter with unspecific binding partners present.

The method described above may be carried out by an analysing system configured to carry out the method. The system may comprise at least an optical readout device such as a microscope configured to receive the sample holder 100 and for generating an optical readout of the sample area 105 and the control area 102, and a liquid handling unit for applying at least the staining reagent to the sample area 105 and the control area 102. Optionally, the analysing system may further comprise a robotic handling unit for handling a plurality of sample holders 100 and shuttling the sample holders 100 between the optical readout device and the liquid handling unit.

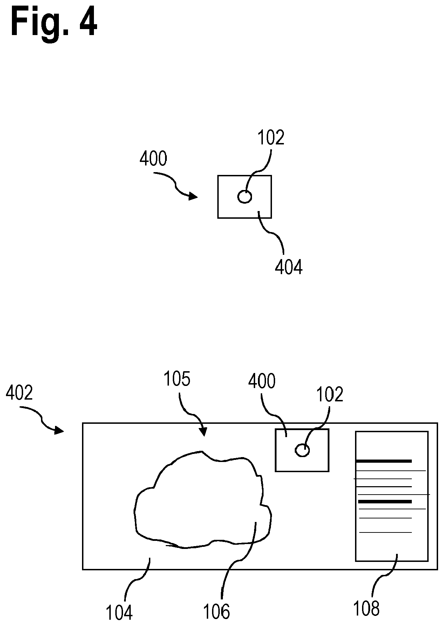

Figure 4 is a schematic top view of a flexible carrier 400 for carrying the control area 102 and a sample holder 402 comprising the flexible carrier 400 with the control area 102. The flexible carrier 400 comprises a first side 400 on which the control area 102 is arranged. A second side opposite the first side 404 of the flexible carrier comprises an adhesive. This enables fixing the flexible carrier 400 on a surface by the adhesive on the second side. For example, the flexible carrier 400 may be fixed on the base 104 of the sample holder 402 in proximity of the biological sample. 106. Thus, instead of arranging the control area 102 directly on the base 104 of the sample holder 402, the control area 102 may easily applied to the base 104 by fixing the flexible carrier 400 with the previously applied control area 102 to the base 104.

The flexible carrier 400 may comprise several control areas 102. In this case, each control area 102 may comprise a specific control analyte and/or each control area 102 may comprise the same control analyte but in different densities. Alternatively, each control area may comprise several different control analytes.

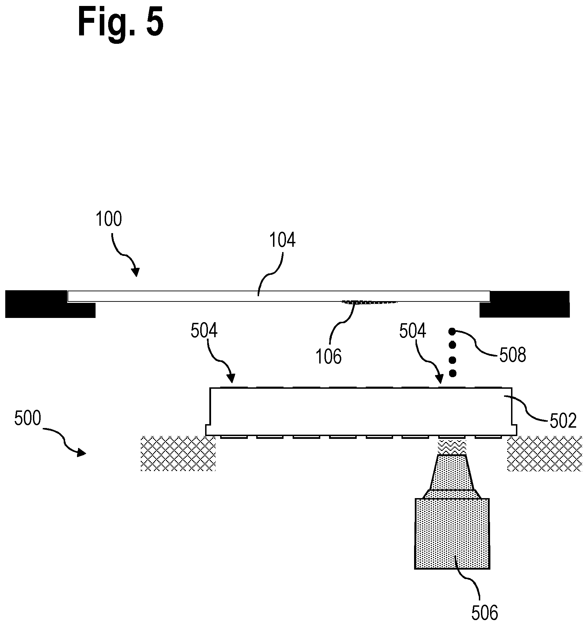

Figure 5 is a schematic side view of a system 500 for generating control areas. For example, the system 500 may generate the control area 102 on the base 104 in order to generate the sample holder 100. The system 500 comprises liquid reservoir in the form of a microtiter plate 502 with several wells 504. Each well 504 of the microtiter plate 502 may comprise a solution comprising at least one particular control analyte. The system further comprises a liquid dispenser 506. The liquid dispenser 506 may be a piezo actuated dispenser, for example. The liquid dispenser 506 is configured to generate droplets 508 of the solution of the wells 504 of the microtiter plate 502 and deposit the droplets on a surface of the base 104 of the sample holder 100. Thus, the liquid dispenser 506 may deposit the solution in form of droplets onto the surface of the base 104 without coming in contact with the surface of the base 104. The droplets preferably have a volume in the range of 1 to 1000 nanolitres. The system 500 may similarly generate control areas on the flexible carrier 400.

At least one of the wells 504 of the microtiter plate 502 may comprise a polymerising reagent solution. The polymerising reagent solution may similarly be deposited onto the surface of the base 104, in particular on the control area 102, for example, to cross-link the control analyte of the control area 102. Further wells 504 may each comprise different control analytes, that may individually be deposited onto the surface of the base 104 in one control area or several individual control areas. Alternatively or additionally, some wells 504 may comprise a solution comprising several control analytes. In order to deposit solutions from different wells 504 onto the surface the liquid dispenser 506 and the microtiter plate 502 may move relative to each other.

The system 500 as well as the flexible carrier 400 may be particularly beneficial for generating control areas on sample holders on which biological samples are already arranged, but which do not comprise control areas or comprise control areas that are not suitable or irrelevant for a particular biological analysis of the biological sample. By means of the system control areas may be generated on sample holders after the biological sample is arranged on the sample holder and the properties of the biological sample may be considered when generating the control areas, for example, to choose the particular control analyte.

The system 500 may carry out a method for generating a control area that includes depositing on a surface a solution comprising the at least one control analyte in order to generate the control area. Preferably, the solution is deposited in the form of droplets. The method may further comprise depositing a polymerising reagent in the control area. To that end, the system 500 may deposit several droplets onto the surface, for example, a droplet of the solution comprising the control analyte and a further droplet comprising the polymerising reagent. The polymerising reagent and the control analyte may then react on the surface and the polymerising reagent may link the control analyte to the surface, for example.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 402: Sample holder
- 102, 102a, 102b, 102c, 102d: Control area
- 104: Base of sample holder
- 105: Sample area
- 106: Biological sample
- 108: Identifier
- 200a, 200b, 200c, 200d: Control analyte
- 202a, 202b, 202c, 202d: Marker
- 400: Flexible carrier
- 404: First side of flexible carrier
- 500: System for generating a control area
- 502: Microtiter plate
- 504: Well
- 506: Liquid dispenser
- 508: Liquid droplet

## Claims

1. A method for generating at least a first control area (102) for a biological analysis, the method comprising the following steps:
depositing on a surface a solution comprising a first control analyte (200a, 200b, 200c, 200d) in order to generate the first control area (102).

2. The method according to claim 1, wherein a polymerising reagent is deposited in the first control area (102).

3. The method according to one of the preceding claims, wherein the solution is deposited on the surface in form of at least one droplet and/or wherein the solution comprises the first control analyte (200a, 200b, 200c, 200d) at a predetermined density.

4. The method according to one of the preceding claims, wherein the first control analyte (200a, 200b, 200c, 200d) is a biomolecule.

5. The method according to one of the preceding claims, wherein a second solution comprising a second control analyte (200a, 200b, 200c, 200d) is deposited on the surface.

6. The method according to one of the preceding claims, wherein the surface is of a base (104) of a sample holder (100, 402).

7. The method according to claim 6, wherein the base (104) of the sample holder (100, 402) comprises a sample area (105) configured to receive a biological sample (106), or wherein the base (104) of the sample holder (100, 402) comprises a sample area (105) with a biological sample (105) and the first control analyte (200a, 200b, 200c, 200d) is identical to a first target analyte of the biological sample (106).

8. The method according to one of the preceding claims 6 or 7, wherein the base (104) of the sample holder (100, 402) comprises a sample area (105) with a biological sample (106) and the biological sample (106) is determined on the base (104) of the sample holder (100, 402) and the solution comprising the first control analyte (200a, 200b, 200c, 200d) is deposited on the surface of the base (104) adjacent to the biological sample (106).

9. The method according to one of the preceding claims 1 to 5, wherein the surface is of a flexible carrier (400) with a first side (404) and a second side opposite the first side (404), wherein the solution comprising the first control analyte (200a, 200b, 200c, 200d) is deposited on the first side (404) of the flexible carrier (400) and the second side comprises an adhesive.

10. The method according to claim 9, wherein the flexible carrier (400) is fixed to a base of a sample holder (100, 402).

11. A method for analysing a biological sample including at least one first target analyte, the method in particular comprises the method steps for generating at least a first control area (102) according to one of the preceding claims, the method comprises the step of providing the biological sample in a sample area (105) of a sample holder (100, 402), the method further comprising the following steps:
a) adding a staining reagent, the staining reagent at least comprising a first optically detectable marker specific to the first target analyte, to the sample area (105) and to at least a first control area (102) of the sample holder (100, 402),
b) generating an optical readout of at least the first control area (102) by means of an optical readout device, and
c) determining for at least the first control area (102) an intensity parameter of at least the first optically detectable marker (202a-202d) in the optical readout.

12. The method according to claim 11 further comprising a step d): generating at least one optical readout of the biological sample (106) in the sample area (105) and analysing the biological sample (106) based on the determined intensity parameter.

13. The method according to one of the preceding claims 11 or 12, wherein the step c) or the step d) comprises rejecting the stained biological sample (106) in case that the intensity parameter is below a predetermined threshold.

14. The method according to one of the claims 11 to 13, wherein the first control area (102) comprises the first control analyte (200a-200d) at a predetermined density, and wherein step c) comprises adjusting at least one optical readout parameter of the optical readout device based on the intensity parameter.

15. The method according to one of the claims 11 to 14, wherein in a step e) at least the first optically detectable marker (202a-202d) is removed or quenched, and optionally wherein the steps a) to e) are iteratively repeated.

16. A system (500) for generating at least a first control area (102) comprising at least a first control analyte (200a, 200b, 200c, 200d) on a surface, or an analysing system for analysing a biological sample (106) comprising at least one first target analyte, the system (500) comprising means configured to carry out the method according to one of the preceding claims.

17. A flexible carrier (400) for carrying at least a first control area (102), comprising
a first side (404) and a second side opposite the first side (404),
wherein the first side (404) of the flexible carrier (400) comprises at least a first control area (102) comprising at least a first control analyte (200a, 200b, 200c, 200d),
wherein the second side of the flexible carrier (400) comprises an adhesive, and
wherein preferably the at least a first control area (102) is generated on the flexible carrier (400) according to the method according to one of the claims 1 to 10.

18. A sample holder (100, 402) for a biological sample (106) including at least one first target analyte, the sample holder comprising:
a sample area (105) configured to hold the biological sample (106), and
at least a first control area (102) comprising at least one first control analyte (200a, 200b, 200c, 200d) identical to the one first target analyte.

19. The sample holder according to claim 18, wherein the at least first control area (102) comprises the first control analyte (200a-200d) at a predetermined density.

20. The sample holder according to one of the preceding claims 18 or 19, comprising an identifier (108), preferably the identifier (108) is optically detectable.

21. The sample holder according to one of the preceding claims 18 to 20, wherein the sample area (105) is spatially separate from at least the first control area (102).

22. The sample holder according to one of the preceding claims 18 to 21, comprising a base (104) on which the sample area (105) and the first control area (102) are arranged and wherein the base (104) is adapted to enable observation of the biological sample (106) by means of an optical readout device, and/or wherein at least a part of the sample holder (100) comprising the sample area (105) is optically transparent.

23. The sample holder according to one of the preceding claims 18 to 22, wherein the at least first control area (102) comprises at least one second control analyte, and/or wherein the sample holder (100) comprises a second control area (102) comprising at least one second control analyte, wherein the first control area is spatially separated from the second control area.
